# FASCICULE DE BREVET EUROPEEN

(11) **EP 3 813 735 B1**
(45) Date de publication et mention de la délivrance du brevet: **17.04.2024**
(21) Numéro de dépôt: 19748577.4
(22) Date de dépôt: 27.06.2019
(51) Int. Cl.: A61F 2/52

(54) **PROTHESE MAMMAIRE EXTERNE AEREE**
BELÜFTETE EXTERNE BRUSTPROTHESE
AERATED EXTERNAL MAMMARY PROSTHESIS

(30) Priorité: 27.06.2018 FR 1855797
(43) Date de publication de la demande: 05.05.2021
(73) Titulaire: Sanchez, Leonarda, 31620 Bouloc (FR)
(72) Inventeur: Sanchez, Leonarda, 31620 Bouloc (FR)
(74) Mandataire: IP Trust
(86) Numéro de dépôt international: PCT/FR2019/051589
(87) Numéro de publication internationale: WO 2020/002843

(56) Documents cités:
- WO-A1-2011/136746
- FR-A1- 2 465 471
- US-A- 401 028
- US-A- 404 881
- US-A- 4 566 458
- US-A- 4 795 464
- US-A1- 2002 016 129

## Description

### DOMAINE DE L'INVENTION / ETAT DE LA TECHNIQUE

L'invention concerne une prothèse mammaire externe destinée à remplacer un sein ayant subi une mastectomie, totale ou partielle, et dont l'aspect et le comportement dynamique se rapproche du sein naturel.

### ETAT DE LA TECHNIQUE

Selon la date à laquelle l'opération de mastectomie a eu lieu, il existe deux types de prothèses.

Le premier type concerne les prothèses mammaires transitoires dites de première intention, lesquelles sont destinées à être portées juste après l'opération, et durant les deux mois suivants, le temps de la cicatrisation. Ces prothèses sont non adhérentes : elles ne se fixent pas directement sur la peau mais sont maintenues plaquées sur le torse par le biais du soutien-gorge à l'intérieur duquel elles sont disposées. Ces prothèses sont classiquement réalisées en textile. Elles se présentent plus particulièrement sous la forme d'un coussinet comprenant une enveloppe en matériau souple textile remplie d'un matériau de rembourrage. Les prothèses connues à ce jour présentent cependant deux inconvénients majeurs. En premier lieu, les matériaux de rembourrage employés, principalement la cellulose, comprennent potentiellement des nanoparticules parmi lesquelles on peut retrouver des perturbateurs endocriniens. En deuxième lieu, lors de leur port, les prothèses initiales sont plaquées contre les plaies de l'opération, ce qui empêche toute aération de ces dernières pendant toute la durée du port et de ce fait ralentit leur cicatrisation.

Le deuxième type concerne les prothèses mammaires dites de deuxième intention, lesquelles sont destinées à être portées généralement à partir du deuxième mois après l'opération. Ces prothèses sont classiquement réalisées en silicone. Elles sont conçues dans la mesure du possible de façon à avoir le même poids et la même apparence que le sein naturel. Afin d'ajuster le poids de la prothèse à l'utilisatrice, il est connu de la demande WO2015197938 de recourir à des éléments de lestage. S'il peut être ainsi obtenu des prothèses ajustées pour remplacer des seins d'un certain volume, il est difficile de proposer des prothèses adaptées pour remplacer des seins menus, la structure même d'une telle prothèse s'avérant d'emblée trop lourde. On connait également du brevet US401028 une prothèse destinée à être appliquée par simple pression sur le torse à l'aide d'une main et tenue en position sur ce dernier par effet ventouse lors du relâchement de la pression. La prothèse en question est constituée d'une coque non perforée et élastique, de forme conique, et d'une structure ressort formée de fils torsadées disposés en surface ou en interne de la coque. La structure ressort constitue ainsi des entretoises de renforcement élastique permettant le déploiement de la coque lorsque la pression appliquée sur celle-ci est relâchée. L'inconvénient d'une telle prothèse est son imperméabilité à l'air. Par ailleurs, la présence de la structure ressort et son arrangement avec la coque empêche tout comportement dynamique de la prothèse qui offre alors un aspect « figé » éloigné de celui d'un sein naturel. Document US 401 028 A et US 404 881 A montrent des prothèses mammaires externes.

L'invention vise à remédier à ces problèmes en proposant une prothèse mammaire externe permettant de palier conjointement les inconvénients des prothèses mammaires initiales et les prothèses mammaires de deuxième intention.

Ainsi l'invention vise à proposer une prothèse mammaire externe dépourvue de nanoparticules pouvant contenir des perturbateurs endocriniens et assurant une aération des plaies de sorte à favoriser la cicatrisation des plaies.

L'invention vise également une prothèse mammaire externe allégée tout en présentant un comportement dynamique proche d'un sein naturel.

L'invention vise également une prothèse mammaire pouvant être portée indifféremment à la suite d'une mastectomie aussi bien totale que partielle.

L'invention vise en outre une prothèse mammaire externe pouvant être mise en oeuvre tant à titre de première intention qu'à titre de deuxième intention.

### OBJET DE L'INVENTION

A cet effet, et selon un premier aspect, l'invention propose une prothèse mammaire externe comprenant une ossature en matériau souple présentant une forme de sein, ladite ossature définissant une cavité interne et présentant en extrémité proximale une base annulaire conformée à la forme du torse d'une personne et en extrémité distale une zone discale pleine présentant la forme d'un mamelon et de son auréole attenante, la base annulaire étant reliée à la zone discale par des bras de forme sinueuse réalisés en matériau flexible, lesdits bras délimitant la cavité interne et étant espacés les uns des autres pour définir des zones d'aération débouchant dans la cavité interne.

En prévoyant une ossature ainsi ouverte, la circulation d'air est favorisée au travers des bras, évitant ainsi le maintien des plaies dans un environnement dans lequel les sécrétions et humidités stagneraient, et favorisant la cicatrisation des plaies en leur permettant de « respirer ». En outre, la forme sinueuse des bras permet une distribution des contraintes sur toute leur longueur, limitant ainsi le risque d'affaissement de l'ossature. La structure de la prothèse mammaire est de surcroit allégée. Enfin, l'existence de l'espace interne délimité par les bras a pour avantage de loger la masse restante du sein en cas de mastectomie partielle, la prothèse mammaire comblant ainsi le vide laissé par l'ablation partielle du sein et offrant un galbe similaire à celui du sein restant.

Avantageusement, les bras sont distribués autour de l'axe sagittal de manière à présenter une forme en spirale.

Avantageusement, les bras sont déformables conjointement en rotation autour de l'axe sagittal et en translation le long de cet axe pour permettre le passage de la prothèse mammaire d'une position dite d'usage dans laquelle les bras sont déployés à une position dite de rangement dans laquelle les bras sont ployés. Le caractère expansible de la prothèse mammaire selon l'invention permet ainsi d'assurer un encombrement optimal au fin de son rangement.

Avantageusement, lorsqu'ils sont en position de rangement, les bras et la zone discale se situent dans un même plan frontal.

Avantageusement, la zone discale est logée, en position de rangement, à l'intérieur de la base annulaire.

Avantageusement, la base annulaire présente une surface de contact avec le torse croissant en direction de la partie supérieure de la prothèse mammaire.

Avantageusement, la cavité interne est fermée au niveau de la base annulaire par un opercule présentant une face adaptée pour être plaquée contre le torse de la personne.

Avantageusement, la prothèse mammaire est formée d'un seul tenant.

### BREVE DESCRIPTION DES FIGURES

D'autres objets et avantages de l'invention apparaîtront au cours de la description qui suit, faite en référence aux dessins annexés, dans lesquels :
- la figure 1 représente une vue schématique de face d'une prothèse mammaire externe selon l'invention ;
- la figure 2 représente une vue schématique arrière de la prothèse mammaire de la figure 1 ;
- la figure 3 représente une vue schématique de côté (côté droit) de la prothèse mammaire de la figure 1 ;
- la figure 4 représente une vue schématique de dessous de la prothèse mammaire de la figure 1 ;
- la figure 5 représente une vue schématique de la prothèse mammaire en position de rangement.

### DESCRIPTION DETAILLEE DES FIGURES

Dans la présente description, on utilise les termes classiquement employés dans le domaine médical en physiologie pour définir les différentes orientations dans l'espace par rapport au corps d'un patient, en l'occurrence le plan frontal ou l'axe sagittal.

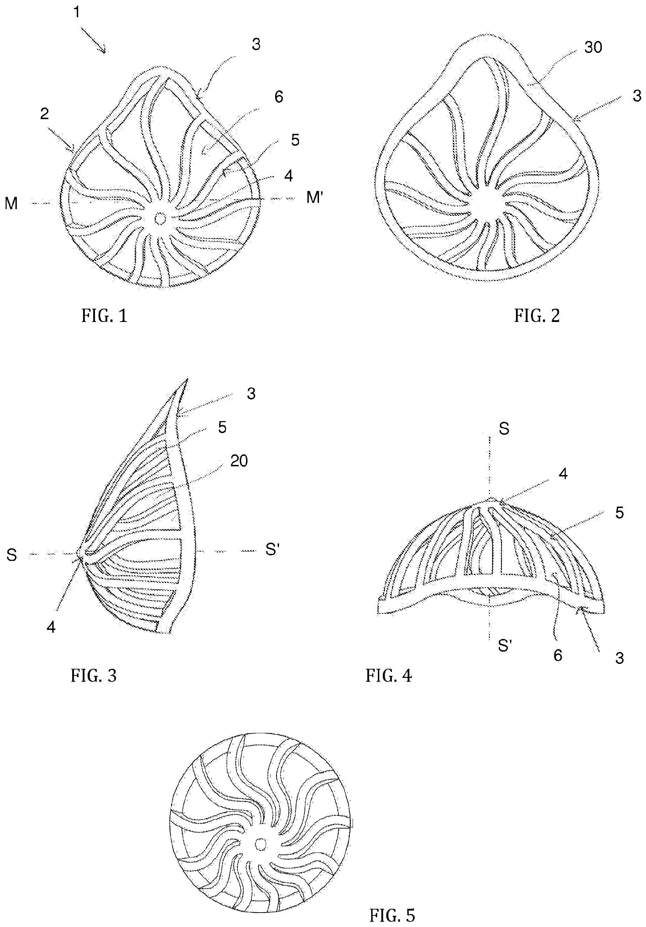

En relation avec les figures 1 à 4, il est décrit un exemple de réalisation d'une prothèse mammaire 1 externe selon l'invention.

La prothèse mammaire 1 comprend une ossature 2 en matériau souple présentant une forme de sein. L'ossature 2 définit une cavité interne 20 présentant, dans l'exemple illustré, une forme de calotte sphérique. L'ossature 2 est préférentiellement réalisée en silicone.

L'ossature 2 selon l'invention comporte, en extrémité proximale, une base annulaire 3 apte à épouser la forme du torse d'une personne et, en extrémité distale, une zone discale 4 pleine présentant la forme d'un mamelon et de son auréole attenante. La base annulaire 3 délimite un espace 30, la zone discale 4 définit un axe sagittal SS' perpendiculaire au plan de la base annulaire 3.

L'ossature 2 comporte en outre des bras 5 réalisés en matériau flexible reliant la base annulaire 3 et la zone discale 4. Les bras 5 forment avec la zone discale 4 et la base annulaire 3 une seule et même pièce. Les bras 5 sont arrangés pour définir avec les bras directement adjacents des zones d'aération 6 débouchant dans la cavité interne 20. La présence de telles zones permet le passage de l'air et favorise ainsi la cicatrisation lorsque la prothèse mammaire 1 selon l'invention est mise en oeuvre dans le cadre d'une prothèse de première intention.

Selon une configuration préférée de l'invention, les bras 5 ont une forme sinueuse et sont distribués autour de l'axe sagittal SS' de manière à présenter une forme spiralée. Dans le mode de réalisation illustré, chaque bras 5 présente une alternance de deux rayons de courbure. Les rayons de courbure de chaque bras peuvent être identiques ou différents Afin de conférer une rigidité suffisante à l'ossature 2, les bras 5 sont montés entre la base annulaire 3 et la zone discale 4 avec une légère torsion.

L'ossature 2 ainsi formée définit une coque de prothèse supportant mieux l'écrasement du poids des habits au regard des prothèses de l'art antérieur d'une part et assurant une répartition optimale du poids et de la pression appliqués sur la prothèse d'autre part.

Du fait de leur distribution autour de la zone distale et de leur caractère flexible, la prothèse mammaire 1 est adaptée pour passer d'une position dite d'usage dans laquelle les bras 5 sont déployés à une position dite de rangement dans laquelle les bras 5 sont ployés comme illustré sur la figure 5. Le passage de la position d'usage à la position de rangement de la prothèse mammaire 1 est obtenu par entrainement des bras 5 suivant un mouvement de rotation autour de l'axe sagittal SS' et par appui au niveau de la zone discale 4 pour entrainer les bras 5 suivant un mouvement de translation le long de ce même axe en direction de l'espace 30. Le passage de la position de rangement à la position d'usage de la prothèse est quant en lui réalisé par relâchement de la force d'appui exercée au niveau de la zone discale 4, les bras 5 se déployant suivant un mouvement de rotation en sens inverse autour de l'axe sagittal et un déplacement en translation le long de cet axe en direction opposé à la base annulaire 3. En position de rangement, les bras 5 et la zone discale 4 se situent dans un même plan frontal, la zone discale 4 étant préférentiellement logée au niveau de l'espace 30 défini par la base annulaire 3 afin d'optimiser l'encombrement.

Afin d'assurer un contact satisfaisant avec le torse de l'utilisateur, la base annulaire 3 présente avantageusement une surface de contact 30 avec le torse plus important en partie supérieure. On définit la partie supérieure de la prothèse mammaire lorsque celle-ci est portée. Elle est délimitée de la partie inférieure par la ligne médiane MM' illustrée sur la figure 1. La figure 2 montre une surface de contact 30 croissant en direction de la partie supérieure de la prothèse mammaire 1. L'avantage de prévoir une surface plus importante est de renforcer la tenue de l'ossature 2.

Avantageusement, la prothèse mammaire 1 comporte un opercule (non représenté) placé au niveau de la base annulaire 3 pour fermer la cavité interne 20 délimitée par l'ossature 2. L'opercule présente une face adaptée pour être plaquée en tout ou partie contre le torse de la personne. Selon une configuration particulière, la face de l'opercule est pourvue d'empreintes arrangées pour correspondre à la forme et au volume des plaies résultant de la mastectomie. L'avantage d'une structure ainsi arrangée est de permettre de réduire toute pression sur la cicatrice. Il peut être prévu également que les empreintes soient ménagées sur une structure additionnelle apte à être fixée de manière amovible sur l'opercule.

La prothèse mammaire externe venant d'être décrite est destinée à être maintenue en place contre le torse à l'aide d'un soutien-gorge, d'une brassière ou équivalent. L'ossature constitue le corps de prothèse en tant que tel et non une structure de soutien à une coque ou tout élément additionnel à l'ossature tel que des élément de rembourrage. En d'autres termes, la prothèse est formée avantageusement de la seule ossature et dont la base est pourvue, le cas échéant, d'un opercule tel que décrit précédemment. Grâce à un tel arrangement, la prothèse selon l'invention est « respirable ».

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Prothèse mammaire (1) externe **caractérisée en ce qu'**elle comprend une ossature (2) en matériau souple présentant une forme de sein, ladite ossature (2) définissant une cavité interne (20) et présentant en extrémité proximale une base annulaire (3) conformée à la forme du torse d'une personne et en extrémité distale une zone discale (4) pleine présentant la forme d'un mamelon et de son auréole attenante, la zone discale (4) définissant un axe sagittal, la base annulaire (3) étant reliée à la zone discale (4) par des bras (5) de forme sinueuse réalisés en matériau flexible, lesdits bras (5) délimitant la cavité interne (20) et étant espacés les uns des autres pour définir des zones d'aération (6) débouchant dans la cavité interne (20).

2. Prothèse mammaire (1) externe selon la revendication 1, **caractérisée en ce que** les bras (5) sont distribués autour de l'axe sagittal de manière à présenter une forme en spirale.

3. Prothèse mammaire (1) externe selon la revendication précédente, **caractérisée en ce que** les bras (5) sont déformables conjointement en rotation autour de l'axe sagittal et en translation le long de cet axe pour permettre le passage de la prothèse mammaire (1) d'une position dite d'usage dans laquelle les bras (5) sont déployés à une position dite de rangement dans laquelle les bras (5) sont ployés.

4. Prothèse mammaire (1) externe la revendication 3, **caractérisée en ce qu'**en position de rangement, les bras (5) et la zone discale (4) se situent dans un même plan frontal.

5. Prothèse mammaire (1) externe la revendication 3 ou la revendication 4, **caractérisée en ce qu'**en position de rangement, la zone discale est logée à l'intérieur de la base annulaire (3).

6. Prothèse mammaire (1) externe selon l'une quelconque des revendications précédentes, **caractérisée en ce que** la base annulaire (3) présente une surface de contact (30) avec le torse croissant en direction de la partie supérieure de la prothèse mammaire (1).

7. Prothèse mammaire (1) externe selon l'une quelconque des revendications précédentes, **caractérisée en ce que** la cavité interne (20) est fermée au niveau de la base annulaire (3) par un opercule présentant une face adaptée pour être plaquée contre le torse de la personne.

8. Prothèse mammaire (1) externe selon l'une quelconque des revendications précédentes, **caractérisée en ce qu'**elle est formée d'un seul tenant.

## Patentansprüche

1. Externe Brustprothese (1), **dadurch gekennzeichnet, dass** sie eine Konstruktion (2) aus flexiblem Material umfasst, die eine Brustform aufweist, wobei die Konstruktion (2) einen inneren Hohlraum (20) definiert und an einem proximalen Ende eine ringförmige Basis (3), die der Form des Torsos einer Person angepasst ist, und an einem distalen Ende einen gefüllten Bandscheibenbereich (4) aufweist, der die Form einer Brustwarze und ihres angrenzenden Vorhofes aufweist, wobei der Bandscheibenbereich (4) eine Sagittalachse definiert, wobei die ringförmige Basis (3) mit dem Bandscheibenbereich (4) durch gewundene Arme (5) aus flexiblem Material verbunden ist, wobei die Arme (5) den inneren Hohlraum (20) begrenzen und voneinander beabstandet sind, um Belüftungsbereiche (6) zu definieren, die in den inneren Hohlraum (20) münden.

2. Externe Brustprothese (1) nach Anspruch 1,
**dadurch gekennzeichnet, dass** die Arme (5) um die Sagittalachse verteilt sind, um eine Spiralform aufzuweisen.

3. Externe Brustprothese (1) nach dem vorstehenden Anspruch,
**dadurch gekennzeichnet, dass** die Arme (5) gemeinsam in Rotation um die Sagittalachse und in Translation entlang dieser Achse verformbar sind, um den Übergang der Brustprothese (1) von einer sogenannten Gebrauchsposition, in der die Arme (5) ausgebreitet sind, in eine sogenannte Unterbringungsposition, in der die Arme (5) gebeugt sind, zu ermöglichen.

4. Externe Brustprothese (1) nach Anspruch 3,
**dadurch gekennzeichnet, dass** in der Unterbringungsposition die Arme (5) und der Bandscheibenbereich (4) in einer gleichen Frontalebene liegen.

5. Externe Brustprothese (1) nach Anspruch 3 oder 4,
**dadurch gekennzeichnet, dass** der Bandscheibenbereich in der Unterbringungsposition innerhalb der ringförmigen Basis (3) eingesetzt ist.

6. Externe Brustprothese (1) nach einem der vorstehenden Ansprüche, **dadurch gekennzeichnet, dass** die ringförmige Basis (3) eine Kontaktoberfläche (30) mit dem Torso aufweist, die in Richtung des oberen Teils der Brustprothese (1) zunimmt.

7. Externe Brustprothese (1) nach einem der vorstehenden Ansprüche, **dadurch gekennzeichnet, dass** der innere Hohlraum (20) an der ringförmigen Basis (3) durch ein Verschlussorgan geschlossen ist, das eine Seite aufweist, die so beschaffen ist, dass sie gegen den Torso der Person gepresst werden kann.

8. Externe Brustprothese (1) nach einem der vorstehenden Ansprüche, **dadurch gekennzeichnet, dass** sie einstückig ausgebildet ist.

## Claims

1. External mammary prosthesis (1), **characterized in that** it comprises a framework (2) of flexible material having the shape of a breast, said framework (2) defining an internal cavity (20) and having, at a proximal end, an annular base (3) conformed to the shape of the chest of a person and, at a distal end, a solid disc zone (4) having the shape of a nipple and its adjoining areola, the disc zone (4) defining a sagittal axis, the annular base (3) being connected to the disc zone (4) by sinuous arms (5) made of flexible material, said arms (5) demarcating the internal cavity (20) and being spaced apart from each other in order to define aeration zones (6) opening into the internal cavity (20).

2. External mammary prosthesis (1) according to claim 1,
**characterized in that** the arms (5) are distributed about the sagittal axis so as to have a spiral shape.

3. External mammary prosthesis (1) according to the preceding claim, **characterized in that** the arms (5) are deformable jointly in rotation about the sagittal axis and in translation along this axis to allow the passage of the mammary prosthesis (1) from a so-called usage position in which the arms (5) are deployed to a so-called storage position in which the arms (5) are folded.

4. External mammary prosthesis (1) according to claim 3,
**characterized in that**, in the storage position, the arms (5) and the disc zone (4) are located in the same frontal plane.

5. External mammary prosthesis (1) according to claim 3 or claim 4, **characterized in that**, in the storage position, the disc zone is housed inside the annular base (3).

6. External mammary prosthesis (1) according to any one of the preceding claims, **characterized in that** the annular base (3) has a contact surface (30) with the chest increasing toward the upper part of the mammary prosthesis (1).

7. External mammary prosthesis (1) according to any one of the preceding claims, **characterized in that** the internal cavity (20) is closed at the annular base (3) by a cover having a face adapted to be pressed against the chest of the person.

8. External mammary prosthesis (1) according to any one of the preceding claims, **characterized in that** it is formed in one piece.
